(19) Europäisches Patentamt
European Patent Office
Office européen des brevets

(11) **EP 3 735 902 B1**

(12) **EUROPEAN PATENT SPECIFICATION**

(45) Date of publication and mention
of the grant of the patent:
**01.12.2021 Bulletin 2021/48**

(51) Int Cl.:
*A61B 5/11* (2006.01)   *A61B 5/00* (2006.01)

(21) Application number: **20167915.6**

(22) Date of filing: **03.04.2020**

(54) **METHOD AND APPARATUS OF DETECTING GAIT PARAMETER THROUGH HEAD PART ACCELERATION SENSOR**

VERFAHREN UND VORRICHTUNG ZUR ERFASSUNG VON GANGPARAMETERN DURCH EINEN KOPFTEILBESCHLEUNIGUNGSSENSOR

PROCÉDÉ ET APPAREIL DE DÉTECTION DE PARAMÈTRE DE DÉMARCHE PAR L'INTERMÉDIAIRE D'UN CAPTEUR D'ACCÉLÉRATION DE PARTIE DE TÊTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.05.2019 KR 20190053477**

(43) Date of publication of application:
**11.11.2020 Bulletin 2020/46**

(73) Proprietor: **Beflex Inc.**
**Daejeon 34104 (KR)**

(72) Inventor: **JUNG, CHANG KEUN**
**34052 Daejeon (KR)**

(74) Representative: **Banzer, Hans-Jörg**
**Kraus & Weisert**
**Patentanwälte PartGmbB**
**Thomas-Wimmer-Ring 15**
**80539 München (DE)**

(56) References cited:
**KR-B1- 101 830 371     US-A1- 2018 279 916**

• **KAWAMURA K ET AL: "Gait phase detection using foot acceleration for estimating ground reaction force in long distance gait rehabilitation", JOURNAL OF ROBOTICS AND MECHATRONICS OCTOBER 2012 FUJI TECHNOLOGY PRESS JPN, vol. 24, no. 5, October 2012 (2012-10), pages 828-837, XP002799235,**
• **DESAILLY E ET AL: "Foot contact event detection using kinematic data in cerebral palsy children and normal adults gait", GAIT & POSTURE, ELSEVIER, AMSTERDAM, NL, vol. 29, no. 1, 1 January 2009 (2009-01-01), pages 76-80, XP025803947, ISSN: 0966-6362, DOI: 10.1016/J.GAITPOST.2008.06.009 [retrieved on 2008-08-03]**
• **HUI ZHOU ET AL: "Towards Real-Time Detection of Gait Events on Different Terrains Using Time-Frequency Analysis and Peak Heuristics Algorithm", SENSORS, vol. 16, no. 10, 1 October 2016 (2016-10-01), page 1634, XP055696823, DOI: 10.3390/s16101634**
• **SANGIL CHOI ET AL: "Biometric gait recognition based on wireless acceleration sensor using k-nearest neighbor classification", 2014 INTERNATIONAL CONFERENCE ON COMPUTING, NETWORKING AND COMMUNICATIONS (ICNC), IEEE, 3 February 2014 (2014-02-03), pages 1091-1095, XP032585862, DOI: 10.1109/ICCNC.2014.6785491**

**Description**

**CROSS-REFERENCE TO RELATED APPLICATIONS**

This application claims priority to Korean Patent Application No. 10-2019-0053477, filed on May 8, 2019, in the Korean Intellectual Property Office.

**TECHNICAL FIELD**

The following disclosure relates to a method and an apparatus of detecting a gait parameter through a head part acceleration sensor, and more particularly, to a method and an apparatus of detecting a gait parameter through a head part acceleration sensor capable of improving analysis accuracy for a gait posture by collecting a gait acceleration signal using an acceleration sensor attached to a head part of a pedestrian to detect a gait point-in-time and a gait parameter.

**BACKGROUND**

Recently, development for a system that analyzes physical activities of a person in daily life using various sensors has been actively conducted. A gait among the physical activities of the person has been known as a natural movement that hardly requires cerebration, but has been found to be actually related to high-level cognitive functions such as concentration and executive ability.

Therefore, a gait analysis of the person has been utilized as an important measure for evaluating whether or not a subject may maintain normal daily life.

An apparatus of measuring a gait motion according to the related art has directly measured ground reaction forces by attaching sensors to limb parts of a human body or has collected ground reaction forces separated from each other from two different force plates. Since the apparatus of measuring a gait motion according to the related art separately collects the ground reaction forces for the left foot and the right foot from the two force plates as described above, there was a disadvantage that an expensive equipment should be purchased in order to analyze a gait posture of a pedestrian. Furthermore, in a case of collecting the ground reaction forces using the force plates, there was a disadvantage that portability is poor.

Conventionally, there was an attempt to measure running using an acceleration sensor. However, since a person moves at a high velocity at the time of the running, only acceleration signals for the left foot and the right foot are mainly measured, and a double-limb support hardly appear. Therefore, it was very difficult to analyze a single-limb support or a separate movement of the left foot or the right foot using an acceleration sensor according to the related art.

**SUMMARY**

In order to solve such a problem in the related art, the present applicant has found that it is possible to analyze a gait posture of a pedestrian by attaching only an acceleration sensor to a head part of the pedestrian without using the force plates through an experiment. In addition, the present applicant has found that in a case of analyzing the gait posture by attaching the acceleration sensor to the head part, accuracy of the analysis is improved more effectively than a case of attaching acceleration sensors to limb parts.

According to the invention, a method of detecting a gait parameter as defined in claim 1 and an apparatus of detecting a gait parameter as defined in claim 6 are provided. The dependent claims 2-5 and 7 define preferred and advantageous embodiments of the invention.

**BRIEF DESCRIPTION OF THE DRAWINGS**

FIGS. 1A to 1B are schematic views illustrating an apparatus of detecting a gait parameter through a head part acceleration sensor according to an exemplary embodiment of the present invention (1A) and according to an example not falling under the scope of the claims (1B)

FIG. 2 is a configuration diagram of the apparatus of detecting a gait parameter according to an exemplary embodiment of the present invention.

FIG. 3 is a schematic flowchart of a method of detecting a gait parameter through a head part acceleration sensor according to an exemplary embodiment of the present invention.

FIG. 4 is a detailed flowchart of the method of detecting a gait parameter according to an exemplary embodiment of the present invention.

FIG. 5 is an illustrative view for describing a process of detecting a gait point-in-time by collecting a gait acceleration signal according to an exemplary embodiment of the present invention.

FIG. 6 is an illustrative view for describing a process of detecting the highest point, the lowest point, and a jerk of the collected gait acceleration signal according to an exemplary embodiment of the present invention.

FIGS. 7 and 8 are illustrative views for describing a process of setting a window on the basis of the highest point of the gait acceleration signal according to an exemplary embodiment of the present invention.

FIG. 9 is an illustrative view for describing a process of detecting a start point and an end point of a double-limb support at a jerk in the window according to an exemplary embodiment of the present invention.

FIG. 10 is an illustrative view for describing a process of distinguishing a left foot and a right foot from each other depending on the gait acceleration signal according to an exemplary embodiment of the present invention.

FIG. 11 is a view for describing a double-limb support and a single-limb support according to an exemplary embodiment of the present invention.

FIG. 12 is a view for describing a process of analyzing a gait posture of a user on the basis of peak values in region A of FIG. 11.

FIG. 13 is an illustrative view illustrating the gait posture of the user through FIG. 12 in detail.

FIGS. 14A and 14B are graphs illustrating ground reaction forces for each time according to another exemplary embodiment of the present invention.

## DETAILED DESCRIPTION OF EMBODIMENTS

In addition, in the following description, ordinal expressions such as first and second are intended to describe objects that are equivalent to and are independent of each other, and should be understood to have no meaning of main/sub or master/slave in the order.

The objects, features, and advantages described above will become more obvious from the following detailed description provided in relation to the accompanying drawings.

Hereinafter, various exemplary embodiments of the present invention will be described in detail with reference to the accompanying drawings.

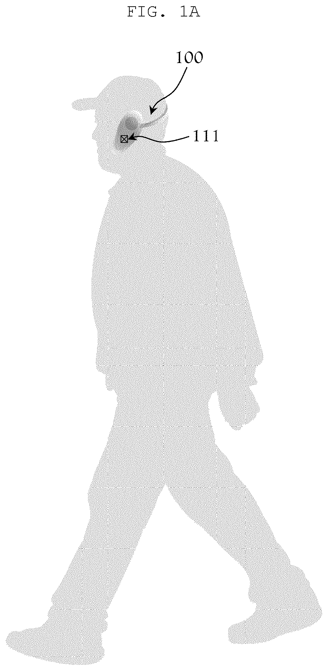

Hereinafter, a configuration of an apparatus 100 of detecting a gait parameter according to an exemplary embodiment of the present invention and a method of detecting a gait parameter using the same will be described with reference to FIGS. 1A to 4.

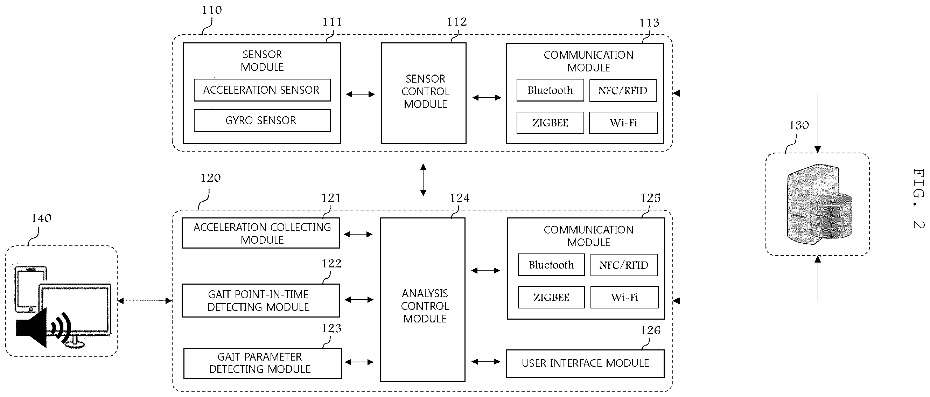

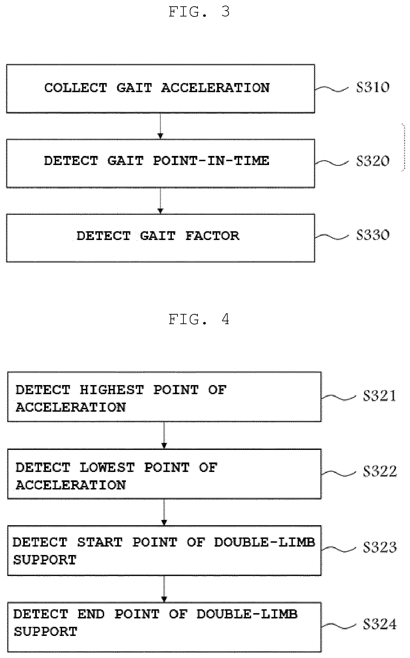

FIGS. 1A to 1B are schematic views illustrating an apparatus of detecting a gait parameter through a head part acceleration sensor according to an exemplary embodiment of the present invention. FIG. 2 is a configuration diagram of the apparatus of detecting a gait parameter according to an exemplary embodiment of the present invention. FIG. 3 is a schematic flowchart of a method of detecting a gait parameter through a head part acceleration sensor according to an exemplary embodiment of the present invention. FIG. 4 is a detailed flowchart of the method of detecting a gait parameter according to an exemplary embodiment of the present invention.

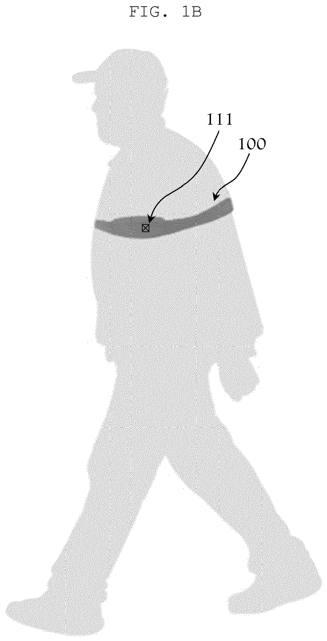

Referring to FIGS. 1A and 1B, an apparatus 100 of detecting a gait parameter is an apparatus that may accurately analyze a gait motion by collecting a gait acceleration of a pedestrian when the pedestrian walks, and is an acceleration sensor attached to a headset, an earphone, a hat, and the like, which may be worn on a head of the pedestrian. In the present invention, the apparatus 100 of detecting a gait parameter is basically attached to the headset to measure the gait parameter, as illustrated in FIG. 1A. However, the apparatus 100 of detecting a gait parameter is not limited thereto, and may be worn on the upper body in the vicinity of the waist or the chest of a user (see FIG. 1B), which, however, is not covered by the scope of the claims.

The apparatus 100 of detecting a gait parameter includes a gait measuring module 110, a gait analyzing module 120, a database 130, and a notifying module 140, as illustrated in FIG. 2.

The gait measuring module 110 measures a gait acceleration signal of the pedestrian using an acceleration sensor attached to a head part of the pedestrian, and is attached to the headset. However, the gait measuring module 110 is not limited thereto, and may be detached from and attached to the chest part of the pedestrian (see FIG. 1B) using a band or be attached to the vicinity of the head or the vicinity of the upper body of the pedestrian, the latter not falling under the scope of the claims. For example, the gait measuring module 110 may be attached to the vicinity of the upper arm, the ear, the head, and the shoulder of the pedestrian using a band or various apparatuses. However, according to the invention, the gait measuring module 110 is attached to a part close to the head part except for the lower body, because accuracy of measurement is decreased in a case where the gait measuring module 110 is attached to and detached from a body part below the upper body. In addition, in the present invention, the gait measuring module 110 may be accommodated in a housing separate from a housing in which the gait analyzing module 120 is accommodated or may be accommodated in the same housing as a housing in which the gait analyzing module 120 is accommodated.

Referring to FIG. 2, the gait measuring module 110 may include a sensor module 111, a sensor control module 112 and a communication module 113.

The sensor module 111 may measure sensor values required for a gait analysis of the gait analyzing module 120 and transmit the measured sensor values to the gait analyzing module 120. It has been described in the present invention that the sensor module 111 senses only the sensor values required for the gait analysis, but the sensor module 111 may detect a gait point-in-time and a gait parameter by collecting a sensed acceleration and transmit the detected gait point-in-time and gait parameter to the gait analyzing module 120. In addition, the sensor module 111 may include an acceleration sensor and a gyro sensor, or may perform measurement using a global positioning system (GPS) sensor. A detailed measuring method related to the sensor module will be described later. In addition, the sensor control module 112 may control a general operation of the gait measuring module 110.

The communication module 113 may transmit the sensor values sensed by the sensor module 111 to the gait analyzing module 120. The communication module 113 may include, for example, a Bluetooth module, a near field communication (NFC) module, a radio frequency identification (RFID) module, a Zigbee module, a wireless fidelity (Wi-Fi) module, and the like.

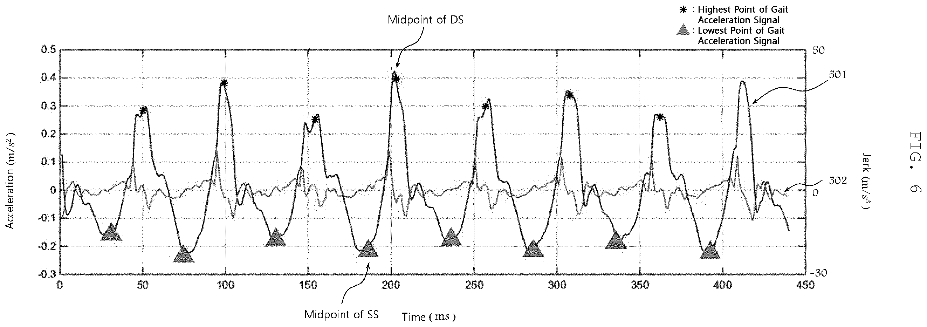

The gait analyzing module 120 includes an acceleration collecting module 121 receiving a gait acceleration signal from the sensor module 111 of the gait measuring module 110 and collecting a gait acceleration, a gait point-in-time detecting module 122 detecting a gait point-in-time by detecting the lowest point and the highest point of the gait acceleration from the collected gait acceleration, and a gait parameter detecting module 123 deriving a gait parameter on the basis of the detected gait point-in-time. In addition, a communication module 125 may be provided in order to transmit a signal to the gait measuring module 110 and the notifying module 140. Detailed operations of the gait point-in-time detecting module 122 and the gait parameter detecting module 123 will be described later with reference to FIGS. 6 to 13.

Meanwhile, in a case where the acceleration collecting module 121 is formed integrally with the gait measuring module 110, the communication module 113 of the gait measuring module 110 may be directly connected to the gait analyzing module 120 to transmit the collected gait acceleration signal to the gait analyzing module 120. In addition, such information on the gait analysis may be output through the notifying module 140. Such information may be output by, for example, a speaker, a mobile phone, a computer, a wireless earphone, or the like.

The notifying module 140 converts gait posture analysis information generated by the gait analyzing module 120 into information that may be recognized by a user, such as a sound or an image, and outputs the converted information. For example, in a case where a stride correction is necessary, the notifying module 140 may output a voice such as "reduce a stride" or a warning sound such as "beep" through a speaker provided in the apparatus 100 of detecting a gait parameter or a mobile phone, a computer, and a wireless earphone connected to the apparatus 100 of detecting a gait parameter. Here, in a case where a stride to be corrected by the user is long, the warning sound is quickly output, and in a case where a stride to be corrected by the user is short, the warning sound is slowly output, such that the user may recognize how much the stride needs to be corrected with only the warning sound and correct the stride. Particularly, it has been mentioned that the wireless earphone or the speaker may be included in the notifying module 140, but the acceleration sensor is basically attached to the earphone worn on the head part of the user in the present invention, and the gait posture analysis information may thus be output directly to ears of the user through the apparatus 100 of detecting a gait parameter. In addition, the notifying module 140 may be realized in various forms, such as being connected to a smart phone, a computer, a dedicated display, or the like, to output accurate correction information as an image.

In addition, the apparatus 100 of detecting a gait parameter may transmit and store the gait posture analysis information derived by the gait analyzing module 120 to and in the database 130. Here, the gait posture analysis information may be cumulatively stored, such that the gait posture of the pedestrian may be confirmed according to a change in time. Therefore, when a large amount of gait posture analysis information is cumulatively stored, such data may be variously utilized. For example, such data may be utilized as big data to be used for various statistics and analyses. An analysis control module 124 of the apparatus 100 of detecting a gait parameter may control a general operation of the gait analyzing module 120 described above.

The apparatus 100 of detecting a gait parameter is attached to the head part of the pedestrian to collect the gait acceleration (S310). Then, the apparatus 100 of detecting a gait parameter detects the gait point-in-time (S320).

The gait point-in-time refers to the highest point or the lowest point of the acceleration or a start point and an end point of a single-limb support or a double-limb support, detected from the gait acceleration signal collected in order to detect the gait parameter. Here, the gait acceleration refers to an acceleration that changes during a period in which the pedestrian walks, and a gait cycle of the pedestrian may be determined on the basis of the gait acceleration. For example, as illustrated in FIG. 4, the gait acceleration of the pedestrian is calculated by measuring each of accelerations depending on the gait cycle.

Referring to FIG. 4, the gait cycle may be divided into a standing phase and a swing phase. The standing phase is a phase in which a body weight is loaded while a foot comes into contact with the ground, and may also be defined as a posture step. The swing phase is a phase in which a limb is away from the ground and moves forward, and may also be defined as a swing step. In this case, the body weight is loaded on the other limb. As illustrated in FIG. 4, the standing phase occupies about 60% of the gait cycle and the swing phase occupies the remaining 40% of the gait cycle.

A section where both feet come into contact with the ground at the same time during the gait cycle is called a double-limb support (DS), and appears twice during one gait cycle. In addition, a section in which only one foot comes into contact with the ground during the gait cycle is called a single-limb support (SS), and appears twice during one gait cycle. In this case, as a gait velocity of the pedestrian become fast, the single-limb support (SS) may be increased and the double-limb support (DS) may be decreased.

Specifically, the standing phase includes gait states of five steps such as a heel strike (HS), a foot flat (FF), a midstand, a heel off (HO), and a toe off (TO). In addition, the swing phase includes gait states of three steps such as a toe off (TO), midswing, and a heel strike (HS). In the present specification, the gait state has been described on the basis of the right foot, and a description of the left foot has been described as an expression such as an opposite side.

In other words, the standing phase includes an initial contact in which the body weight is loaded while the foot starts to come into contact with the ground, a loading response, a midstance, and a terminal stance in which the body weight is loaded while the feet comes into complete contact with the ground, and a pre-swing, which is a phase immediately before the heel of the foot is lifted from the ground while the body weight is loaded on an opposite limb before entering the swing phase.

The swing phase is divided into an initial swing, a midswing and a terminal swing. Specifically, the initial swing is a phase which starts with a toe off and in which the limb moves forward while acceleration is applied to the limb. The midswing is a phase in which the knee joint is extended and the muscle hardly acts as the limb passes through the opposite limb. The terminal swing may also be defined as a velocity reducing period, and in the terminal swing, and may move on to the initial contact of the standing phase by naturally reducing a velocity of the limb moving forward.

Times required for the respective gait phases described above are constant for each individual, but as a pace becomes faster, the double-limb support may be reduced.

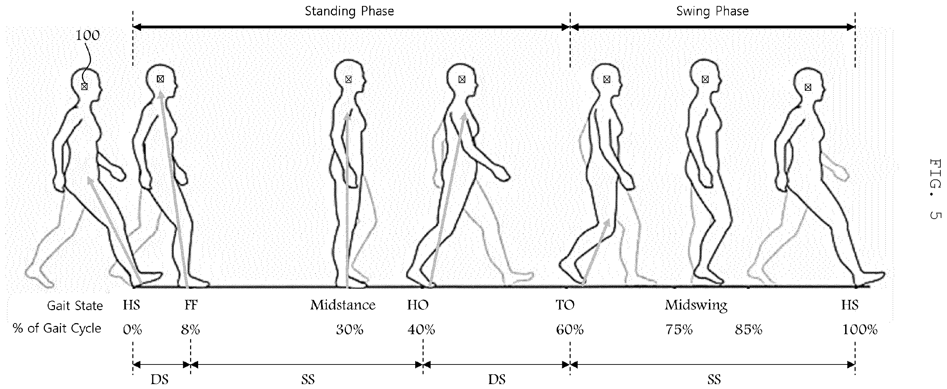

Hereinafter, a process of detecting the gait point-in-time and the gait parameter will be described with reference to FIG. 4 together with FIGS. 5 to 10.

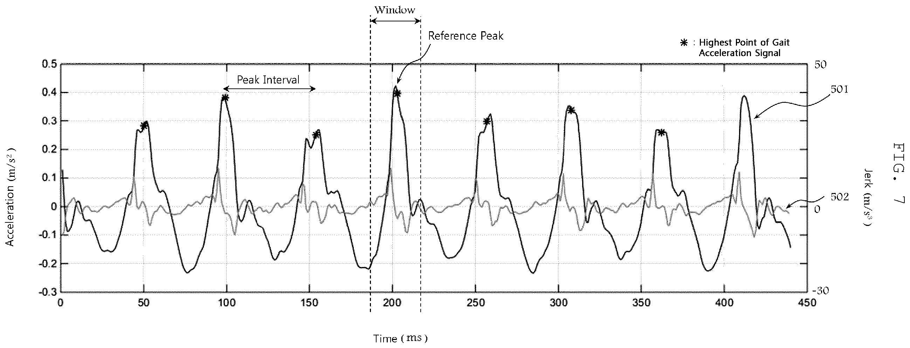

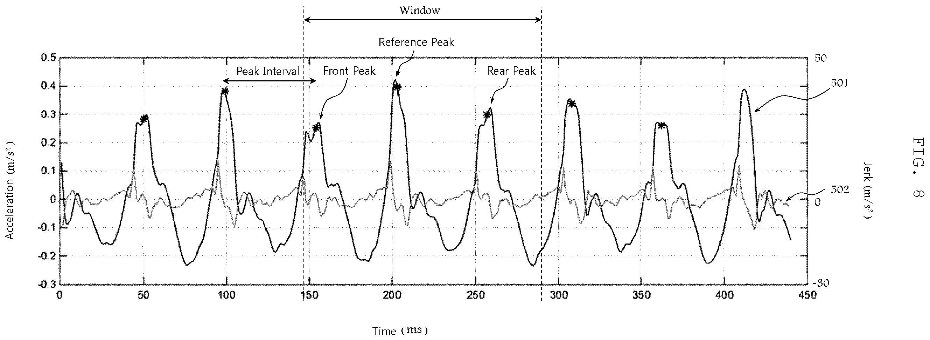

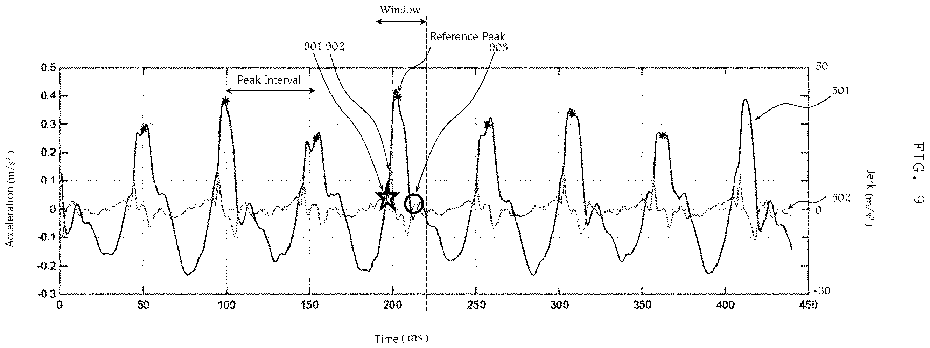

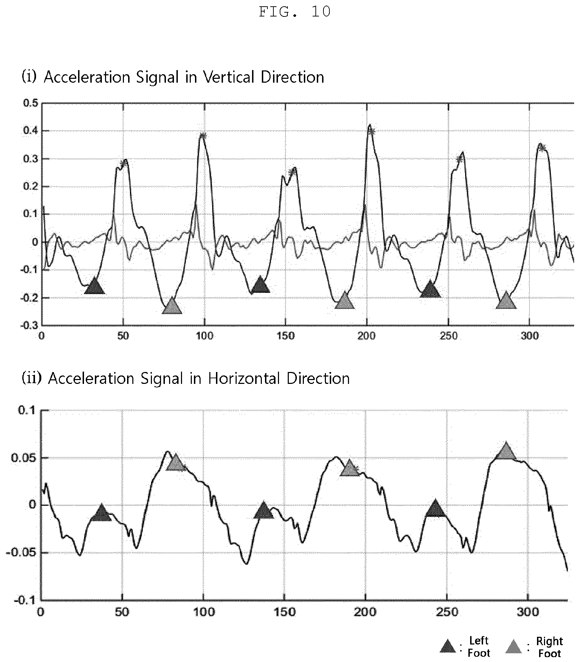

FIG. 5 is an illustrative view for describing a process of detecting a gait point-in-time by collecting a gait acceleration signal according to an exemplary embodiment of the present invention. FIG. 6 is an illustrative view for describing a process of detecting the highest point, the lowest point, and a jerk of the collected gait acceleration signal according to an exemplary embodiment of the present invention. FIGS. 7 and 8 are illustrative views for describing a process of setting a window on the basis of the highest point of the gait acceleration signal according to an exemplary embodiment of the present invention. FIG. 9 is an illustrative view for describing a process of detecting a start point and an end point of a double-limb support at a jerk in the window according to the invention. FIG. 10 is an illustrative view for describing a process of distinguishing a left foot and a right foot from each other depending on the gait acceleration signal according to an exemplary embodiment of the present invention.

The gait analyzing module 120 may detect the gait point-in-time of the left foot and the right foot measured by the head part acceleration sensor attached to the head part of the pedestrian on the basis of the collected gait acceleration signal. Specifically, as illustrated in FIGS. 4 and 6, in order to detect the gait point-in-time (S320), the highest point of the acceleration is detected (S321), and the lowest point of the acceleration is detected (S322). Then, the start point of the double-limb support is detected (S323). Then, the end point of the double-limb support is detected (S324).

Specifically, a gait acceleration signal 501 according to a time (ms) is collected using the apparatus 100 of detecting a gait parameter attached to the head part of the pedestrian when the pedestrian walks. In this case, the gait acceleration signal may be an acceleration in a vertical direction, or may be a norm of a signal of one or more axes including the acceleration in the vertical direction. For example, a one-axis (vertical) acceleration, a multi-axis acceleration including a vertical acceleration, or the like may be used. In this case, parameters that may be collected at the time of using the one-axis acceleration are as illustrated in Table 1, and parameters that may be collected at the time of using the multi-axis acceleration are as illustrated in Table 2.

<Table 1>

| Parameter | Unit |
|---|---|
| Cadence | steps/min |
| Stride Duration | ms |
| First peak acceleration | G |
| Trough acceleration | G |
| Second peak acceleration | G |

(continued)

| Parameter | Unit |
|---|---|
| First peak force (if mass known) | N |
| Trough force (if mass known) | N |
| Second peak force (if mass known) | N |
| Step count | step |
| Vertical oscillation | m |

<Table 2>

| Parameter | Unit |
|---|---|
| Left Single Support Duration | ms |
| Left Double Support Duration (Left trailing limb) | ms |
| Left First peak acceleration | G |
| Left Trough acceleration | G |
| Left Second peak acceleration | G |
| Left First peak force | N |
| Left Trough force | N |
| Left Second peak force | N |
| Right Single Support Duration | ms |
| Right Double Support Duration (Right trailing limb) | ms |
| Right First peak acceleration | G |
| Right Trough acceleration | G |
| Right Second peak acceleration | G |
| Right First peak force | N |
| Right Trough force | N |
| Right Second peak force | N |
| Head angle | deg |
| Step Width | m |

Then, peaks *, which are the highest points in the collected gait acceleration signal 501, are detected, and peaks ▲, which are the lowest points in the collected gait acceleration signal 501, are detected. A peak *, which is the highest value of the peaks *, which are the highest points of the gait acceleration signal 501, indicates a midpoint of the double-limb support (DS), and a peak ▲, which is the lowest value of the peaks ▲, which are the lowest points of the gait acceleration signal 501, indicates a midpoint of the single-limb support (SS).

In addition, by obtaining a gait jerk signal 502, which is a differential value of the gait acceleration signal 501, it is possible to detect a point-in-time in which an impact is applied to the ground when the limb comes into contact with the ground during moving from the single-limb support (SS) to the double-limb support (DS). In addition, the gait jerk signal 502 is necessarily required in order to analyze the gait acceleration signal 501 in more detail. Here, the gait jerk signal 502 may be obtained by detecting peak values in a high pass filter (HPF) extracting a preset high frequency signal, calculating an average value of the detected peak values, and multiplying the average value by a prediction coefficient to calculate a predicted value of a gradient (IVLR) of a vertical ground reaction force.

Here, a unit of the IVLR may be N/s, which is an absolute unit indicating a force per unit time, or may be (body weight (BW) or g)/s, which is a relative unit. Since the sensor according to the present invention basically collects acceleration data rather than a force, data is collected in BW/s, which is a relative unit. When g/s is multiplied by a mass (m) of the user, the IVLR may also be expressed in N/s ((N = m*g)/s), which is an absolute unit. In this case, the IVLR may be included in a prediction coefficient (k1 or k2) or may be calculated by prediction coefficient (k1 or k2) × mass (m) of user as a separate coefficient.

Here, the high frequency signal may refer to an acceleration vertical signal having a frequency of 10 Hz or more. An acceleration vertical signal having a frequency of 5Hz or more is hardly generated by an arbitrary movement of the user and an acceleration vertical signal generated in a situation such as an impact or the like generally exists in a high band. Therefore, in the present invention, the high frequency signal having the frequency of 10Hz or more is intended to be used to predict the gradient (IVLR) of the vertical ground reaction force. However, the present invention is not limited thereto, and high frequency signal bands extracted by filtering may be set to be different from each other and be applied to the present invention, if necessary.

Then, a window size is set before detecting the gait parameter. The window size may mean that a certain section is determined temporally among all samples corresponding to the entire gait acceleration signal 501. A method for determining a gait cycle according to the present invention ends in a window set for real-time application. Specifically, referring to FIG. 7, a peak is repeated every 50 ms, and it may thus be seen that a peak interval is approximately 50 ms. In this case, a window of n seconds before and after a reference peak *, which is the highest point of the gait acceleration signal 501, is set. In this case, the window may be set to be increased or decreased in proportion to the peak interval (a reciprocal of a cadence and approximately 50 ms) (see FIG. 6). For example, the window size may be set to a value obtained by multiplying the peak interval by 0.6. However, it is preferable that the window size is fixed to a number of 0.1 seconds or more and 0.4 seconds or less, which is the double-limb support.

However, it is to be noted that the window size is too large to be set to include a front peak and a rear peak, as illustrated in FIG. 8.

Therefore, according to the invention where it is assumed that the window is set before and after the reference peak as illustrated in FIG. 9, a peak 901 (☆) of the gait jerk signal 502 appearing before the reference peak within a window range is set as the start point of the double-limb support (DS) (or the end point of the single-limb support (SS)). In addition, a peak 903 (o) of the gait jerk signal 502 appearing after the reference peak is set as the end point of the double-limb support (DS) (or the start point of the single-limb support (SS)). In this case, an inflection point 902 of the gait jerk signal exists between the peak 901 (☆) of the gait jerk signal 502 and the reference peak. At the infection point 902, a change amount in an acceleration value may be rapidly reduced.

In addition, the present invention is based on collecting the acceleration signal in the vertical direction at the time of collecting the gait acceleration, but as illustrated in (i) and (ii) of FIG. 10, an acceleration signal in a horizontal direction may be collected together at the time of collecting the acceleration signal in the vertical direction. Therefore, in the present invention, it is possible to distinguish the left foot and the right foot of the pedestrian from each other according to signs of the acceleration signal in the horizontal direction corresponding to peaks ▲ and ▲, which are the lowest points of the acceleration signal in the vertical direction. For example, a case where the sign of the acceleration signal in the horizontal direction corresponding to the acceleration signal in the vertical direction is negative (-) may mean the left foot, and a case where the sign of the acceleration signal in the horizontal direction corresponding to the acceleration signal in the vertical direction is positive (+) may mean the right foot. However, the present invention is not limited thereto, and a case where the sign of the acceleration signal in the horizontal direction corresponding to the acceleration signal in the vertical direction is negative (-) may mean the right foot and a case where the sign of the acceleration signal in the horizontal direction corresponding to the acceleration signal in the vertical direction is positive (+) may mean the left foot.

In addition, as illustrated in (ii) of FIG. 10, a step width in the horizontal direction may be calculated using sizes of points corresponding to peaks ▲ and A of the acceleration signal in the horizontal direction.

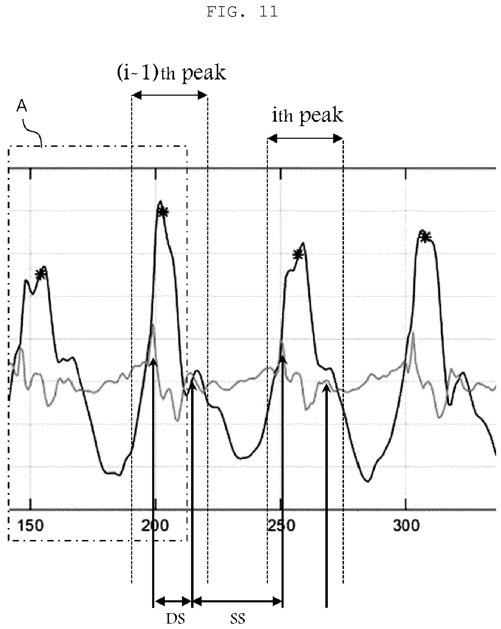

Hereinafter, a process of detecting a gait parameter will be described with reference to FIGS. 11 to 13.

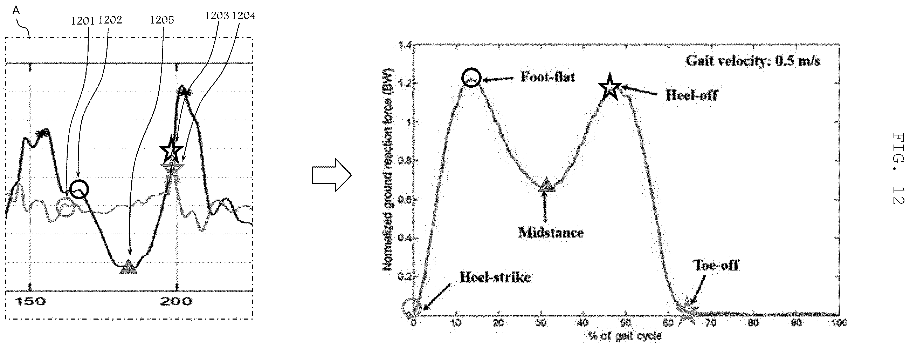

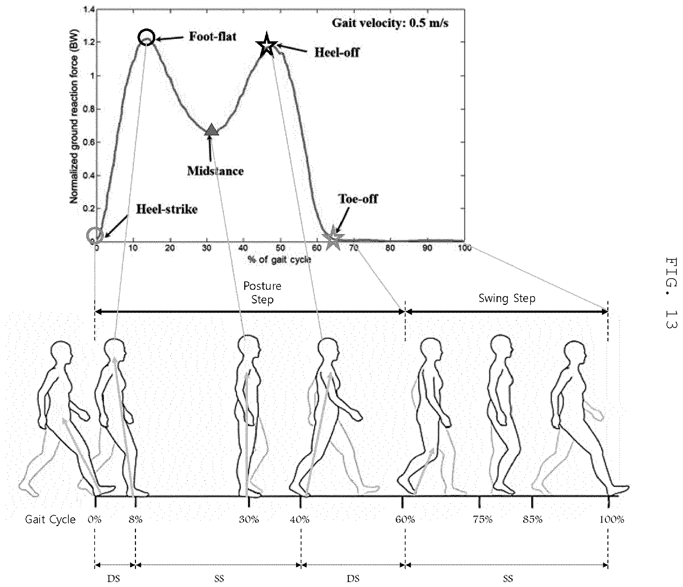

FIG. 11 is a view for describing a double-limb support and a single-limb support according to an exemplary embodiment of the present invention. FIG. 12 is a view for describing a process of analyzing a gait posture of a user on the basis of peak values in region A of FIG. 11. FIG. 13 is an illustrative view illustrating the gait posture of the user through FIG. 12 in detail.

Then, the apparatus 100 of detecting a gait parameter detects the gait parameter (S330). Here, the gait parameter is a component detected in order to analyze the gait posture of the pedestrian on the basis of the detected gait point-in-time, and includes the double-limb support (DS), the single-limb support (SS), a cadence, and a step width. However, the gait parameter is not limited thereto, and may also include force magnitudes of a first peak and a second peak, and a trough force magnitude of the single-limb support within the gait cycle. Therefore, the gait parameter may indicate various components capable of analyzing the gait posture of the pedestrian.

First, referring to FIG. 11, a peak section of the gait jerk signal 502 before and after a peak ((i-1)-th peak) located in the vicinity of a point-in-time of 200 ms is a double-limb support (DS), and a section from a peak of the gait jerk signal after the peak ((i-1)-th peak) to a peak of the gait jerk signal before a peak (i-th peak) located in the vicinity of a point-in-time of 250 ms is a single-limb support (SS). In other words, a time ($t_{DS\,i}$) of a double-limb support of the i-th peak is a value obtained by subtracting a time of a start time of the double-limb support from a time of an end point of the double-limb support (DS) (t DS i = t DS end i - t DS start i). In addition, a time of a single-limb support of the i-th peak is a value obtained by subtracting a time of a start point of the single-limb support from a time of an end point of the single-limb support (t SS i = t SS end i - t SS start i).

In this case, as illustrated in FIG. 11, a time of a start point of an i-th single-limb support is the same as that of an end point of an i-1-th double-limb support (t SS start i = t DS end (i-1)), and a time of an end point of the i-th single-limb support is the same as a time of a start point of an i-th double-limb support (t SS end i = t DS start i).

Referring to FIG. 12, region A is a region in which a section of 150 to 200 ms of the gait cycle is cut, and the gait posture of the pedestrian may be analyzed using peak values located in region A.

Specifically, a first peak acceleration value 1202 indicates a point (foot-flat) at which the foot comes into contact with the ground, and is the highest acceleration value between a time 1201 (t SS start i) of a start point of the i-th single-limb support and a time 1205 (t SS mid i) of an i-th intermediate load section. In addition, a second peak acceleration value 1203 indicates a point (heel-off) at which the heel of the foot is away from the ground, and is the highest acceleration value between the time 1205 (t SS mid i) of an i-th intermediate load section and a time 1204 (t SS end i) of an end point of the i-th single-limb support. In addition, a trough acceleration value indicates a mid stance point, and is an acceleration value corresponding to t SS mid i. In this case, it is possible to find out a force value at a corresponding point-in-time by multiplying the respective values (the first peak acceleration value, the second peak acceleration value, and the trough acceleration value) by a body weight of the pedestrian.

Therefore, as illustrated in FIG. 13, the gait posture of the user may be divided using a graph illustrating a normalized ground reaction force (BW) for each gait cycle (% of gait cycle).

In addition, in FIG. 12, a cadence of the pedestrian may be calculated by taking '1/peak time interval'. However, the cadence may be calculated by various methods. For example, the cadence may be calculated by taking '1/trough time interval', may be calculated by taking '1/time interval between start points of double-limb supports', or may be calculated by taking '1/time interval between end points of double-limb supports'.

Generally, a system of measuring a gait motion according to the related art has collected ground reaction forces by attaching sensors to limb parts of a human body or has collected ground reaction forces separated from each other from two different force plates. Therefore, ground reaction forces for each of the left foot and the right foot are collected, and thus, a work of summing the ground reaction forces for each of the left foot and the right foot needs to be performed in order to analyze the gait posture of the pedestrian. Therefore, there was a disadvantage that work efficiency is low. Furthermore, in a case of collecting the ground reaction forces using the force plates, there was a disadvantage that portability is poor.

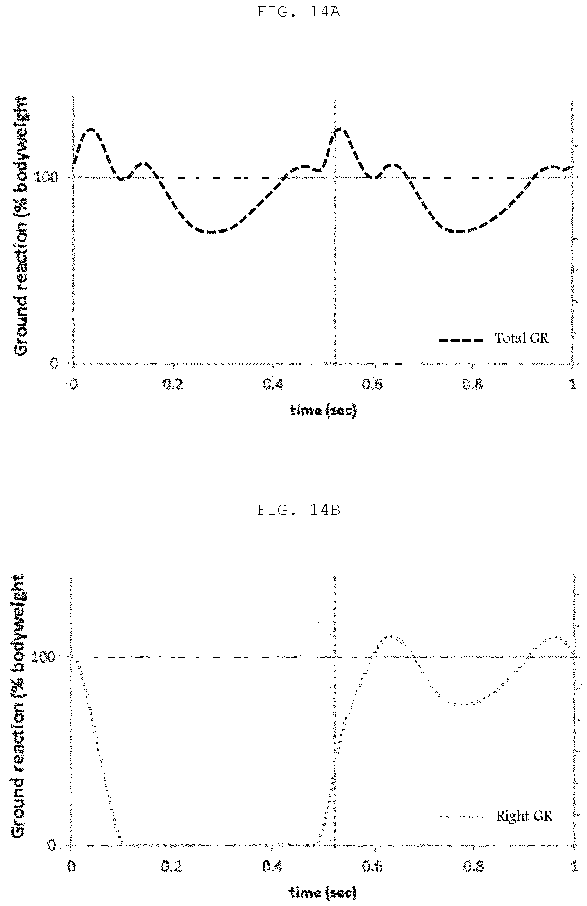

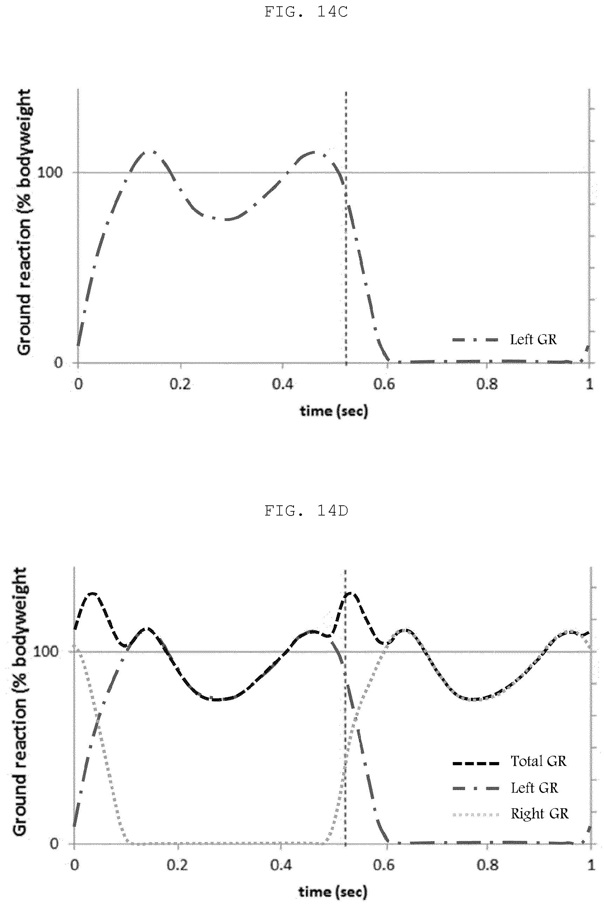

On the other hand, the present applicant has found that it is possible to analyze a gait posture of a pedestrian by attaching only an acceleration sensor to a head part of the pedestrian without using the force plates through an experiment. As illustrated in FIGS. 14A to 14D, it is possible to find the ground reaction forces of each of the right foot and the left foot using a case where the left foot and the right foot come into contact with the ground at the same time (see FIGS. 14B and 14C) after collecting a total ground reaction force (GR) of both feet through the apparatus 100 of detecting a gait parameter attached to the head part (see FIG. 14A). In addition, by using the method of detecting a gait parameter described above, it is also possible to distinguish gait motions from each other such as which section is the single-limb support and which section is the double-limb support from graphs as illustrated in FIG. 14D.

In addition, in the present invention, only the head part acceleration sensor (that is, the apparatus 100 of detecting a gait parameter) is used to collect the gait acceleration signal 501 of the pedestrian and analyze the gait posture of the pedestrian, and it is thus possible to improve a working speed.

In addition, in the present invention, gait pointsin-time for the left foot and the right foot of the pedestrian are distinguished from each other on the basis of the gait acceleration collected from the acceleration sensor attached to the head part of the pedestrian, and it is thus possible to increase convenience of the work.

Further, in the present invention, the vertical acceleration and/or the horizontal acceleration are collected using the apparatus 100 of detecting a gait parameter, such that it is possible to analyze a gait motion in more detail, and it is thus possible to accurately detect an abnormal gait motion of patients with Parkinson's disease or patients with Alzheimer's disease. Further, it is possible to prevent an accident of the patients with Parkinson's disease or the patients with Alzheimer's disease in advance by detecting abnormal gait motion patterns of the patients with Parkinson's disease or the patients with Alzheimer's disease. For example, it is possible to prevent a fall accident or the like by analyzing abnormal gait motion patterns of the patients.

According to the present invention, the head part acceleration sensor attached to the head part of the pedestrian is used to collect the gait acceleration signal of the pedestrian and analyze the gait posture of the pedestrian, and it is thus possible to improve a working speed.

In addition, according to the disclosure, the left foot and the right foot of the pedestrian are distinguished from each other within the gait cycle on the basis of the gait acceleration collected from the acceleration sensor attached to the head part of the pedestrian, and it is thus possible to increase convenience of the work.

The effects according to the present invention are not limited by the contents exemplified in the above, and more various effects are included in the present specification. The invention is only defined by the appended claims.

[Detailed Description of Main Elements]

| | | |
|---|---|---|
| 100: apparatus of detecting gait parameter measuring module | 110: | gait |
| 111: sensor module control module | 112: | sensor |
| 113, 125: communication module analyzing module | 120: | gait |
| 121: acceleration collecting module point-in-time detecting module | 122: | gait |
| 123: gait parameter detecting module control module | 124: | analysis |
| 126: user interface module | 130: | database |
| 140: notifying module acceleration signal | 501: | gait |
| 502: gait jerk signal of gait jerk signal | 901, 903: | peak |

**Claims**

1.   A method of detecting a gait parameter through a head part acceleration sensor, comprising:

a gait acceleration collecting step (S310) of collecting an acceleration (501) in a vertical direction of a pedestrian measured by an acceleration sensor disposed at a head part of the pedestrian;
a gait point-in-time detecting step (S320) of setting a gait point-in-time by extracting a peak value in the acceleration (501) in the vertical direction within a gait cycle including a standing phase and a swing phase and detecting a start point and an end point of a double-limb support on the basis of the peak value; and
a gait parameter detecting step (S330) of detecting the double-limb support on the basis of the start point and the end point in order to analyze a gait posture of the pedestrian,
wherein the start point and the end point of the double-limb support are set within a window predetermined on the basis of peaks of the gait cycle, the start point of the double-limb support is the same as an end point (1204) of a single-limb support, and the end point of the double-limb support is the same as a start point (1201) of a single-limb support
**characterized in that**
in the gait point-in-time detecting step (S320), a gait jerk signal (502) at a point-in-time in which an impact is applied to a ground when a limb moves from the single-limb support to the double-limb support is further detected by differentiating the acceleration (501) in the vertical direction, and
a peak value (901) of the gait jerk signal (502) located before the peak value of the acceleration (501) in the vertical direction is set as the start point of the double-limb support, and a peak value (903) of the gait jerk signal (502) located after the peak value of the acceleration (501) in the vertical direction is set as the end point of the double-limb support.

2.   The method of detecting a gait parameter through a head part acceleration sensor of claim 1, wherein in the gait parameter detecting step (S330), a cadence and a step width are detected on the basis of the peak value of the acceleration (501) in the vertical direction, and
a gait velocity, a stride length, and a step length of the pedestrian are further detected in a case of using a global positioning system.

3.   The method of detecting a gait parameter through a head part acceleration sensor of claim 2, wherein the stride length is calculated by the following equation:

$$\mathtt{Stride\ Length\ =\ Gait\ Velocity/Cadence.}$$

4.   The method of detecting a gait parameter through a head part acceleration sensor of any one of claims 1-3, wherein the gait acceleration collecting step (S310) includes a step of collecting an acceleration in a horizontal direction of the pedestrian, and
a left foot and a right foot of the pedestrian are distinguished from each other according to signs of the acceleration in the horizontal direction.

5. The method of detecting a gait parameter through a head part acceleration sensor of any one of claims 1-4, wherein in the window, after a highest peak of the peaks of the gait cycle is set as a reference peak, a window size is set to be smaller than a range between peaks disposed on both sides of the reference peak.

6. An apparatus (100) of detecting a gait parameter through a head part acceleration sensor, comprising:

a gait measuring module (110) configured to measure an acceleration (501) in a vertical direction by a gait acceleration sensor disposed at a head part of a pedestrian and to transfer a measured value; and a gait analyzing module (120) including an acceleration collecting module (121) configured to collect the measured value received from the gait measuring module (110), a gait point-in-time module (122) configured to extract a peak value in the acceleration (501) in the vertical direction on the basis of the measured value and to detect a start point and an end point of a double-limb support, and a gait parameter detecting module (123) configured to detect the double-limb support on the basis of the start point and the end point, wherein the start point and the end point of the double-limb support are set within a window predetermined on the basis of peaks of a gait cycle, the start point of the double-limb support is the same as an end point (1204) of a single-limb support, and the end point of the double-limb support is the same as a start point (1201) of a single-limb support, **characterized in that**
the gait point-in-time module (122) is further configured to detect a gait jerk signal (502) at a point-in-time in which an impact is applied to a ground when a limb moves from the single-limb support to the double-limb support by differentiating the acceleration (501) in the vertical direction, and
to set a peak value (901) of the gait jerk signal (502) located before the peak value of the acceleration (501) in the vertical direction as the start point of the double-limb support, and a peak value (903) of the gait jerk signal (502) located after the peak value of the acceleration (501) in the vertical direction as the end point of the double-limb support.

7. The apparatus of detecting a gait parameter through a head part acceleration sensor of claim 6, further comprising a notifying module (140) configured to convert the gait parameter detected by the gait analyzing module (120) into a form recognized by a user, including a sound or an image, and to output gait posture analysis information so that the pedestrian corrects a gait posture.

**Patentansprüche**

1. Verfahren zum Erfassen eines Gangparameters durch einen Kopfteil-Beschleunigungssensor, umfassend:

einen Gangbeschleunigung-Erfassungsschritt (S310) zum Erfassen einer Beschleunigung (501) in einer vertikalen Richtung eines Fußgängers, welche durch einen Beschleunigungssensor, welcher an einem Kopfteil des Fußgängers angeordnet ist, gemessen wird;
einen Gangzeitpunkts-Erfassungsschritt (S320) zum Festlegen eines Gangzeitpunkts, indem ein Spitzenwert in der Beschleunigung (501) in der vertikalen Richtung innerhalb eines Gangzyklus extrahiert wird, welcher eine Stehphase und eine Schwungphase aufweist, und indem ein Startpunkt und ein Endpunkt einer Doppel-Glied-Unterstützung abhängig von dem Spitzenwert erfasst wird; und
einen Gangparameter-Erfassungsschritt (S330) zum Erfassen der Doppel-Glied-Unterstützung abhängig von dem Startpunkt und dem Endpunkt, um eine Ganghaltung des Fußgängers zu analysieren, wobei der Startpunkt und der Endpunkt der Doppel-Glied-Unterstützung innerhalb eines Fensters gesetzt sind, welches abhängig von Spitzen des Gangzyklus vorbestimmt wird, wobei der Startpunkt der Doppel-Glied-Unterstützung derselbe ist wie ein Endpunkt (1204) einer Ein-Glied-Unterstützung, und wobei der Endpunkt der Doppel-Glied-Unterstützung derselbe ist wie ein Startpunkt (1201) einer Ein-Glied-Unterstützung; **dadurch gekennzeichnet, dass**
in dem Gangzeitpunkt-Erfassungsschritt (S320) ein Gangrucksignal (502) zu einem Zeitpunkt, zu welchem ein Auftreffen auf einen Boden erfolgt, wenn sich ein Glied von der Ein-Glied-Unterstützung zu der Doppel-Glied-Unterstützung bewegt,
darüber hinaus erfasst wird, indem die Beschleunigung (501) in der vertikalen Richtung abgeleitet wird, und wobei ein Spitzenwert (901) des Gangrucksignals (502), welcher vor dem Spitzenwert der Beschleunigung (501) in der vertikalen Richtung liegt, als der Startpunkt der Doppel-Glied-Unterstützung festgelegt wird, und wobei ein Spitzenwert (903) des Gangrucksignals ( 502), welcher nach dem Spitzenwert der Beschleunigung (501) in der vertikalen Richtung liegt, als der Endpunkt der Doppel-Glied-Unterstützung festgelegt wird.

2. Verfahren zum Erfassen eines Gangparameters durch einen Kopfteil-Beschleunigungssensor nach Anspruch 1, wobei in dem Gangparameter-Erfassungsschritt (S330) eine Schrittfrequenz und eine Schrittweite abhängig von dem Spitzenwert der Beschleunigung (501) in der vertikalen Richtung erfasst werden, und
wobei im Falle einer Verwendung eines globalen Positionierungssystems darüber hinaus eine Ganggeschwindigkeit, eine Doppelschrittlänge und eine Schrittlänge des Fußgängers erfasst werden.

3. Verfahren zum Erfassen eines Gangparameters durch einen Kopfteil-Beschleunigungssensor nach Anspruch 2, wobei die Doppelschrittlänge durch die folgende Gleichung berechnet wird:

$$\text{Doppelschrittlänge} = \text{Ganggeschwindigkeit} / \text{Schrittfrequenz}.$$

4. Verfahren zum Erfassen eines Gangparameters durch einen Kopfteil-Beschleunigungssensor nach einem der Ansprüche 1 bis 3, wobei der Gangbeschleunigung-Erfassungsschritt (S310) einen Schritt eines Erfassens einer Beschleunigung in einer horizontalen Richtung des Fußgängers aufweist, und wobei ein linker Fuß und ein rechter Fuß des Fußgängers voneinander gemäß Vorzeichen der Beschleunigung in der horizontalen Richtung unterschieden werden.

5. Verfahren zum Erfassen eines Gangparameters durch einen Kopfteil-Beschleunigungssensor nach einem der Ansprüche 1 bis 4, wobei in dem Fenster, nachdem eine höchste Spitze der Spitzen des Gangzyklus als eine Referenzspitze festgelegt wird, eine Fenstergröße so festgelegt wird, dass sie kleiner ist als ein Bereich zwischen Spitzen, welche auf beiden Seiten der Referenzspitze angeordnet sind.

6. Vorrichtung (100) zum Erfassen eines Gangparameters durch einen Kopfteil-Beschleunigungssensor, umfassend:

ein Gangmessmodul (110), welches ausgestaltet ist, um eine Beschleunigung (501) in einer vertikalen Richtung durch einen Gangbeschleunigungssensor zu messen, welcher an einem Kopfteil eines Fußgängers angeordnet ist, und um einen Messwert zu übertragen; und
ein Ganganalysemodul (120), welches aufweist ein
Beschleunigungserfassungsmodul, welches ausgestaltet ist, um den von dem Gangmessmodul (110) erhaltenen Messwert zu erfassen (121), ein Gangzeitpunktmodul (122), welches ausgestaltet ist, um einen Spitzenwert in der Beschleunigung (501) in der vertikalen Richtung abhängig von dem Messwert zu extrahieren und um einen Startpunkt und einen Endpunkt einer Doppel-Glied-Unterstützung zu erfassen, und ein Gangparameter-Erfassungsmodul (123),
welches ausgestaltet ist, um die Doppel-Glied-Unterstützung abhängig von dem Startpunkt und dem Endpunkt zu erfassen,
wobei der Startpunkt und der Endpunkt der Doppel-Glied-Unterstützung innerhalb eines Fensters gesetzt sind, welches abhängig von Spitzen eines Gangzyklus vorbestimmt wird, wobei der Startpunkt der Doppel-Glied-Unterstützung derselbe ist wie ein Endpunkt (1204) einer Ein-Glied-Unterstützung, und der Endpunkt der Doppel-Glied-Unterstützung derselbe ist wie ein Startpunkt (1201) einer Ein-Glied-Unterstützung,
**dadurch gekennzeichnet, dass**
das Gangzeitpunktmodul (122) darüber hinaus ausgestaltet ist, um ein Gangrucksignal (502) zu einem Zeitpunkt zu erfassen, zu welchem ein Auftreffen auf einen Boden erfolgt, wenn sich ein Glied von der Ein-Glied-Unterstützung zu der Doppel-Glied-Unterstützung bewegt, indem die Beschleunigung (501) in der vertikalen Richtung abgeleitet wird, und um einen Spitzenwert (901) des Gangrucksignals (502), welcher sich vor dem Spitzenwert der Beschleunigung (501) in der vertikalen Richtung befindet, als den Startpunkt der Doppel-Glied-Unterstützung und einen Spitzenwert (903) des Gangrucksignals (502), welcher sich nach dem Spitzenwert der Beschleunigung (501) in der vertikalen Richtung befindet, als den Endpunkt der Doppel-Glied-Unterstützung festzulegen.

7. Vorrichtung zum Erfassen eines Gangparameters durch einen Kopfteil-Beschleunigungssensor nach Anspruch 6, welche darüber hinaus ein Meldemodul (140) umfasst, welches so ausgestaltet ist, dass es den durch das Ganganalysemodul (120) erfassten Gangparameter in eine von einem Benutzer erkannte Form, einschließlich eines Tons oder eines Bildes, umwandelt und eine Ganghaltungsanalyseinformation ausgibt, so dass der Fußgänger eine Ganghaltung korrigiert.

**Revendications**

1.  Procédé de détection d'un paramètre de démarche par l'intermédiaire d'un capteur d'accélération sur une partie de tête, comprenant :

    une étape de collecte d'accélération de démarche (S310) consistant à collecter une accélération (501) dans une direction verticale d'un piéton mesurée par un capteur d'accélération disposé au niveau d'une partie de tête du piéton ;
    une étape de détection de point temporel de démarche (S320) consistant à définir un point temporel de démarche en extrayant une valeur de crête dans l'accélération (501) dans la direction verticale dans un cycle de démarche comportant une phase debout et
    une phase de balancement et en détectant un point de départ et un point d'arrivée d'un appui sur double membre sur la base de la valeur de crête ; et
    une étape de détection de paramètre de démarche (S330) consistant à détecter l'appui sur double membre sur la base du point de départ et du point d'arrivée pour analyser une posture de démarche du piéton,
    le point de départ et le point d'arrivée de l'appui sur double membre étant définis dans les limites d'une fenêtre prédéterminée sur la base des crêtes du cycle de démarche,
    le point de départ de l'appui sur double membre étant le même qu'un point d'arrivée (1204) d'un appui sur un seul membre, et le point d'arrivée de l'appui sur double membre étant le même qu'un point de départ (1201) d'un appui sur un seul membre **caractérisé en ce que**
    à l'étape de détection d'un point temporel de démarche (S320), un signal de saccade de démarche (502) au niveau d'un point temporel où un impact est appliqué à un sol lorsqu'un membre passe de l'appui sur un seul membre à l'appui sur double membre est en outre détecté en différenciant l'accélération (501) dans la direction verticale, et
    une valeur de crête (901) du signal de saccade de démarche (502) située avant la valeur de crête de l'accélération (501) dans la direction verticale est définie comme point de départ de l'appui sur double membre, et une valeur de crête (903) du signal de saccade de démarche (502) située après la valeur de crête de l'accélération (501) dans la direction verticale est définie comme point d'arrivée de l'appui sur deux membres.

2.  Procédé de détection d'un paramètre de démarche par l'intermédiaire d'un capteur d'accélération sur une partie de tête selon la revendication 1, à l'étape de détection de paramètre de démarche (S330), une cadence et une largeur de pas étant détectées sur la base de la valeur de crête de l'accélération (501) dans la direction verticale, et une vitesse de démarche, une longueur d'enjambée et une longueur de pas du piéton étant en outre détectées dans le cas de l'utilisation d'un système de positionnement global.

3.  Procédé de détection d'un paramètre de démarche par l'intermédiaire d'un capteur d'accélération sur une partie de tête selon la revendication 2, la longueur de pas étant calculée par l'équation suivante :

$$\text{Longueur d'enjambée = Vitesse de démarche / Cadence.}$$

4.  Procédé de détection d'un paramètre de démarche par l'intermédiaire d'un capteur d'accélération sur une partie de tête selon l'une quelconque des revendications 1 à 3, l'étape de collecte d'accélération de démarche (S310) comportant une étape de collecte d'une accélération dans une direction horizontale du piéton, et
    un pied gauche et un pied droit du piéton se distinguant l'un de l'autre selon des signes de l'accélération dans la direction horizontale.

5.  Procédé de détection d'un paramètre de démarche par l'intermédiaire d'un capteur d'accélération sur une partie de tête selon l'une quelconque des revendications 1 à 4, dans la fenêtre, après qu'une crête la plus élevée des crêtes du cycle de démarche est définie comme crête de référence, une taille de fenêtre étant définie pour être plus petite qu'une plage entre les crêtes disposées des deux côtés de la crête de référence.

6.  Appareil (100) de détection d'un paramètre de démarche par l'intermédiaire d'un capteur d'accélération sur une partie de tête, comprenant :

    un module de mesure de démarche (110) configuré pour mesurer une accélération (501) dans une direction verticale par un capteur d'accélération de démarche disposé au niveau d'une partie de tête d'un piéton et pour transférer une valeur mesurée ; et

un module d'analyse de démarche (120) comportant un module de collecte d'accélération (121) configuré pour collecter la valeur mesurée reçue à partir du module de mesure de démarche (110), un module de point temporel de démarche (122) configuré pour extraire une valeur de crête dans l'accélération (501) dans la direction verticale sur la base de la valeur mesurée et pour détecter un point de départ et un point d'arrivée d'un appui sur double membre, et un module de détection de paramètre de démarche (123) configuré pour détecter l'appui sur double membre sur la base du point de départ et du point d'arrivée,

le point de départ et le point d'arrivée de l'appui sur double membre étant définis dans les limites d'une fenêtre prédéterminée sur la base des crêtes d'un cycle de démarche,

le point de départ de l'appui sur double membre étant le même qu'un point d'arrivée (1204) d'un appui sur un seul membre, et le point d'arrivée de l'appui sur double membre étant le même qu'un point de départ (1201) d'un appui sur un seul membre,

**caractérisé en ce que**

le module de point temporel de démarche (122) est en outre configuré pour détecter un signal de saccade de démarche (502) au niveau d'un point temporel où un impact est appliqué à un sol lorsqu'un membre passe de l'appui sur un seul membre à l'appui sur double membre en différenciant l'accélération (501) dans la direction verticale, et

définir une valeur de crête (901) du signal de saccade de démarche (502) située avant la valeur de crête de l'accélération (501) dans la direction verticale comme point de départ de l'appui sur double membre, et une valeur de crête (903) du signal de saccade de démarche (502) située après la valeur de crête de l'accélération (501) dans la direction verticale comme point d'arrivée de l'appui sur double membre.

7. Appareil de détection d'un paramètre de démarche par l'intermédiaire d'un capteur d'accélération sur une partie de tête selon la revendication 6, comprenant en outre un module de notification (140) configuré pour convertir le paramètre de démarche détecté par le module d'analyse de démarche (120) en une forme reconnue par un utilisateur, comportant un son ou une image, et pour émettre des informations d'analyse de posture de démarche de sorte que le piéton corrige une posture de démarche.

FIG. 1A

FIG. 1B

FIG. 2

FIG. 3

| COLLECT GAIT ACCELERATION | S310 |

↓

| DETECT GAIT POINT-IN-TIME | S320 |

↓

| DETECT GAIT FACTOR | S330 |

FIG. 4

| DETECT HIGHEST POINT OF ACCELERATION | S321 |

↓

| DETECT LOWEST POINT OF ACCELERATION | S322 |

↓

| DETECT START POINT OF DOUBLE-LIMB SUPPORT | S323 |

↓

| DETECT END POINT OF DOUBLE-LIMB SUPPORT | S324 |

FIG. 5

FIG. 6

FIG. 7

FIG. 8

FIG. 9

FIG. 10

(i) Acceleration Signal in Vertical Direction

(ii) Acceleration Signal in Horizontal Direction

▲ : Left Foot    ▲ : Right Foot

FIG. 11

FIG. 12

FIG. 13

FIG. 14A

FIG. 14B

FIG. 14C

FIG. 14D

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020190053477